# EUROPEAN PATENT APPLICATION

(11) **EP 4 624 586 A1**
(43) Date of publication of application: **01.10.2025**
(21) Application number: 23894795.6
(22) Date of filing: 05.10.2023
(51) Int. Cl.: C12Q 1/04, C12Q 1/06

(54) **METHOD FOR DETERMINING MICROORGANISM WITH PLASTIC DEGRADATION ACTIVITY**

(30) Priority: 21.11.2022 KR 20220156611; 04.10.2023 KR 20230131708
(71) Applicant: Repla Inc., Gyeongsan-si, Gyeongsangbuk-do 38541 (KR)
(72) Inventor: CHOI, Donggeon, Suwon-si Gyeonggi-do 16694 (KR); KIM, Hongrae, Suwon-si Gyeonggi-do 16679 (KR); LEE, Chaerin, Gunsan-si Jeollabuk-do 54098 (KR)
(74) Representative: Maiwald GmbH
(86) International application number: PCT/KR2023/015306
(87) International publication number: WO 2024/111865

(57) **Abstract**

The present invention relates to a method for determining a microorganism with plastic degradation activity by using a redox indicator and a determination kit, and detects a change in color of the redox indicator so as to rapidly and simply determine whether a corresponding microorganism exhibits plastic degradation activity.

## Description

### [Technical Field]

The present invention relates to a method of quickly and easily identifying microorganisms having plastic-degrading activity using a redox indicator and a kit for identifying such microorganisms.

### [Background Art]

The use of plastics has rapidly increased due to their light weight, high physical and chemical durability, high processability, and very low prices, and the amount of plastic production has significantly increased from 234 million tons in 2000 to 460 million tons in 2019. In addition, the amount of plastic waste generated during the same period increased more than two-fold from 156 million tons to 353 million tons (Korea Institute for International Economic Policy (KIEP) World Economic Focus, Vol. 5, No. 13, Current Status and Implications of International Plastic Regulations; published on May 9, 2022).

Due to COVID-19 lockdowns, global plastic usage decreased by 2.2% in 2020 compared to the previous year, but plastic use is increasing again as lockdowns are lifted around the world, particularly in plastic packaging in the fields of healthcare, personal hygiene plastic products, and e-commerce.

The recycling rate of plastic waste worldwide is only 9%, and unrecycled waste plastic is being disposed of through landfill (50%), illegal dumping (22%), and incineration (19%). In particular, plastics account for 80% of marine waste, and marine plastic waste is expected to increase from 9 to 14 million tons per year in 2016 to 23 to 37 million tons per year in 2040. Environmental pollution caused by the influx of plastics not only threatens the ecosystem and human health, but also causes additional costs for waste disposal and recovery from pollution.

Accordingly, research on plastic-degrading microorganisms is being actively conducted as part of methods for treating plastic waste, and these studies begin with identifying plastic-degrading microorganisms. However, the current identification methods for plastic-degrading microorganisms require a lot of resources and labor. Specifically, the current identification method is carried out by culturing microorganisms in a minimal medium containing plastic as a nutrient, measuring the plastic degradation rate and the growth characteristics of the microorganisms, and comprehensively determining the chemical changes of the plastic using analytical equipment (Fourier-transform infrared spectroscopy (FT-IR), nuclear magnetic resonance (NMR), X-ray photoelectron spectroscopy (XPS), gel permeation chromatography (GPC)) *(*Environmental Science & Technology, 49(20), 12087-12093; Frontiers in Marine Science, 7, 567126; and Polymers, 12(1), 123).

However, this method takes an average experimental period of 30 to 60 days and requires a lot of resources and labor. Therefore, a simpler and faster method for identifying plastic-degrading microorganisms is needed.

Meanwhile, there is a patent for a kit that simply identifies microorganisms with agriculturally useful activity using an indicator, but the agriculturally useful activity refers to the microorganism's auxin production ability, denitrification ability, urea solubilization ability, and poorly soluble phosphate solubilization ability, and is unrelated to plastic-degrading activity (Korean Patent Publication No. 10-2018-0101792).

### [Disclosure]

### [Technical Problem]

Under the above-described circumstances, the present inventors conducted research on a method for quickly identifying plastic-degrading microorganisms, and developed a method for quickly identifying plastic-degrading microorganisms using a redox indicator.

Therefore, an object of the present invention is to provide a method of identifying and isolating novel plastic-degrading microorganisms.

### [Technical Solution]

To achieve the above-described object, one aspect of the present invention provides a method of identifying a microorganism having plastic-degrading activity, including:
(a) a step of culturing a microorganism whose plastic-degrading activity is to be confirmed in a minimal medium containing a redox indicator and a plastic; and
(b) a step of confirming a color change of the redox indicator.

According to one embodiment of the present invention, the method may further include (c) a step of determining the microorganism as having plastic-degrading activity when the color of the redox indicator changes.

The "redox indicator" refers to an indicator whose solution exhibits different colors in an oxidized state and a reduced state and thus enables the confirmation of the presence or absence of an oxidation/reduction reaction. The redox indicator used in the present invention may change color when energy generated when a microorganism degrades a plastic is transferred in the form of electrons.

More specifically, the redox indicator may be selected from the group consisting of 2,6-dichlorophenolindophenol (DCPIP), methylene green (MG), and thionine, and preferably DCPIP.

In the present invention, the plastic may be one or more selected from the group consisting of polyethylene terephthalate (PET), polyvinyl chloride (PVC), polystyrene (PS), polypropylene (PP), polyurethane (PU), and polyethylene (PE). In an example of the present invention, the energy generated when a plastic-degrading microorganism degrades PE was confirmed using a redox indicator.

The technical feature of the present invention is to quickly and easily confirm whether a microorganism has plastic-degrading activity by confirming the energy generated when the microorganism degrades a plastic as a color change of a redox indicator, and the type of plastic that the microorganism degrades does not affect the technical feature. Therefore, even when only the degradation of PE by a microorganism is confirmed in an example, the method of identifying a microorganism having plastic-degrading activity of the present invention may be applied regardless of the type of plastic.

Meanwhile, the fact that a microorganism degrades a plastic means that the microorganism produces and secretes a plastic-degrading enzyme. Therefore, the method of identifying a microorganism having plastic-degrading activity of the present invention may also be performed using a culture solution of the microorganism.

More specifically, another aspect of the present invention provides a method of identifying a microorganism having plastic-degrading activity, including:
(a) a step of adding a culture medium of a microorganism whose plastic-degrading activity is to be confirmed to a minimal medium containing a redox indicator and a plastic;
(b) a step of confirming a color change of the redox indicator; and
(c) a step of determining the microorganism as having plastic-degrading activity when the color of the redox indicator changes.

The description of the redox indicator and the plastic is the same as that described in the method of identifying a microorganism having plastic-degrading activity using a microorganism.

Still another aspect of the present invention provides a composition for identifying a microorganism having plastic-degrading activity or a kit for identifying a microorganism having plastic-degrading activity, including a redox indicator and plastic powder.

In the present invention, the composition or/and the kit may be provided in a ready-to-use form containing a redox indicator and plastic powder in each well of a well plate. In addition, the composition or/and the kit may further include a minimum medium, which is a powder or liquid required for microbial culture, and the composition or the kit may be provided in a state in which the redox indicator and minimum medium are mixed.

In an embodiment of the present invention, a minimum medium containing a plastic in powder form and a redox indicator was divided into a well plate, and microorganisms were inoculated and cultured therein. As a result of the culture, it was confirmed that a color change of the redox indicator occurred when a plastic-degrading microorganism was inoculated.

### [Advantageous Effects]

According to the method of identifying a microorganism having plastic-degrading activity according to the present invention, microorganisms having degradation activity for various types of plastics can be discovered quickly and easily, and as a result, they can contribute to solving the problem of plastic waste.

### [Description of Drawings]

FIG. 1 shows the results of confirming the change in the absorbance of a redox indicator while culturing each of a microorganism having plastic-degrading activity and a microorganism without plastic-degrading activity in a minimal medium containing a redox indicator and polyethylene (PE) powder added: At = absorbance measured by culturing a microorganism with PE powder; Act = absorbance measured by culturing only a microorganism without plastic.
FIG. 2 shows the color change of the redox indicator when culturing each of a microorganism having plastic-degrading activity and a microorganism without plastic-degrading activity in a minimal medium containing a redox indicator and PE powder.
FIG. 3 shows the results of measuring the plastic-degrading ability after culturing a microorganism that exhibited a color change of the redox indicator in a minimal medium containing PE powder.
FIG. 4 shows the results of confirming the viable cell count of a microorganism with plastic-degrading ability on day 0 and day 7, while applying the method of identifying a microorganism with plastic-degrading activity: The error bars represent the standard deviation of the mean value.
FIG. 5 shows photographs of an example of a plastic-degrading microorganism screening kit. After adding plastic powder and a minimal medium to each well, each of a microorganism having plastic-degrading activity and a microorganism without plastic-degrading activity was inoculated, and the results are shown in the photographs as the color change of the redox indicator.

### [Modes of the Invention]

Hereinafter, one or more specific embodiments are described in more detail through examples. However, these examples are provided to illustrate one or more embodiments, and the scope of the present invention is not limited to these examples.

### Example: Identification of plastic-degrading microorganisms

### 1-1. Selection of plastic-degrading microorganisms using redox indicators

A microorganism with plastic-degrading activity (*Acinetobacter guillouiae* repla2), discovered by the present inventors, and a microorganism without plastic-degrading activity (*E. coli*) were each cultured in a nutrient medium (Luria-Bertani broth, LB). After culturing for a certain period of time, 1 mL of the culture solution was collected and centrifuged (13,000 rpm, 5 min). The supernatant was discarded, and only the cells were separated and washed twice with 0.9% saline solution. The cells, from which all nutrients had been removed by washing, were used in subsequent experiments.

A microorganism with plastic-degrading activity and a microorganism without plastic-degrading activity were each calculated to have a cell number of 10⁷ and inoculated into a minimal medium. The minimal medium composition used in the experiment was as follows: pH 6.51, 0.7 g NH₂PO₄, 0.7 g K₂HPO₄, 0.7 g MgSO₄·TH₂O, 1.0 g NH₄NO₃, 0.005 g NaCl, 0.002 g FeSO₄•7H₂O, 0.002 g ZnSO₄•7H₂O, 0.001 g MnSO₄•H₂O based on 1 L of distilled water. In addition, polyethylene (PE) (low-density polyethylene, LDPE; Mw ~4,000, Sigma Aldrich) powder was added to the minimal medium at a concentration of 8 g/L, and 2,6-dichlorophenolindophenol (DCPIP) was added as a redox indicator and then the strain with plastic-degrading activity and the strain without plastic-degrading activity were cultured. The color of DCPIP changes from blue to colorless when it receives energy generated from the metabolism of a microorganism in the form of electrons. Through this, it can be easily determined whether the microorganism is a plastic-degrading strain capable of utilizing a plastic as a nutrient.

The microorganism with plastic-degrading activity and the microorganism without plastic-degrading activity were cultured under two conditions: 1) minimal medium + microorganism; and 2) minimal medium + microorganism + LDPE powder. In addition, the absorbance of the medium from 2) was divided by the result from 1) to exclude the metabolic ability of the microorganisms themselves in the minimal medium.

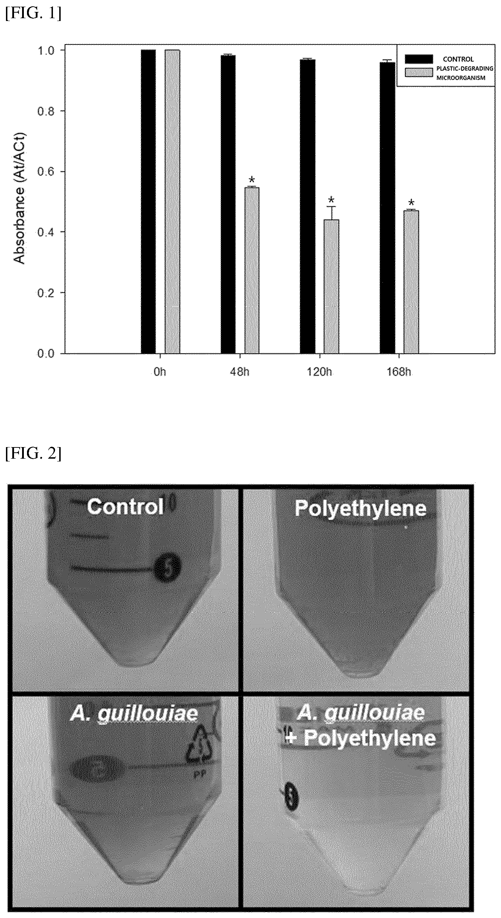

As a result of measuring the absorbance of the medium after culturing the microorganisms for seven days, it was found that the absorbance decreased in the case of the microorganism with plastic-degrading activity, but it did not decrease in the case of the microorganism without plastic-degrading activity. The results showing a statistically significant difference compared to the microorganism without plastic-degrading activity are marked with an asterisk (*) (Mann-Whitney U test, p <0.05) (FIG. 1).

In FIG. 1, "At" indicates the absorbance measured by culturing the microorganisms with PE powder, and "Act" indicates the absorbance measured by culturing only microorganisms without a plastic. The results are expressed as At/Act, and the At/Act value of 1 means that the microorganism does not have plastic-degrading ability because the metabolic activity is not different from that in the minimal medium.

In addition, when photographs were taken after seven days of culture, the color change of the redox indicator could be visually confirmed when the plastic-degrading microorganism was cultured under the minimal medium + microorganism + LDPE powder condition (*A*. *guillouiae* + Polyethylene in FIG. 2). However, the redox indicator exhibited no color change when each of the minimal medium (Control), LDPE powder + minimal medium (Polyethylene), and the plastic-degrading microorganism (*A*. *guillouiae*) was cultured (FIG. 2).

### 1-2. Verification of plastic degradation activity of microorganism confirmed by color change of redox indicator

The plastic degradation rate of the microorganism was measured according to the presence or absence of a color change of the redox indicator. 25 mL of minimal medium and 0.2 g of LDPE powder were added to a 50 mL tube, and a strain with plastic-degrading activity and a strain without plastic-degrading activity were washed and inoculated. They were cultured in a shaking incubator at 120 rpm and 25 °C for 7, 14, and 28 days.

After culturing for the above-mentioned periods, a certain amount of the culture medium was recovered, and the recovered culture medium was sieved through a 40 µm sieve to collect the LDPE powder. To remove the microorganism from the collected LDPE powder, a 2% surfactant and the LDPE powder were allowed to react for 24 hours. Thereafter, the LDPE powder was washed with distilled water, dried at 60 °C for 24 hours, and the weight of the LDPE powder was measured. The plastic degradation rate was calculated according to the following Mathematical Formula 1. Plastic degradation rate (%) = (Initial weight of LDPE powder in the medium - Weight of LDPE powder measured after culturing with microorganism) / (Initial weight of LDPE powder in the medium*100)

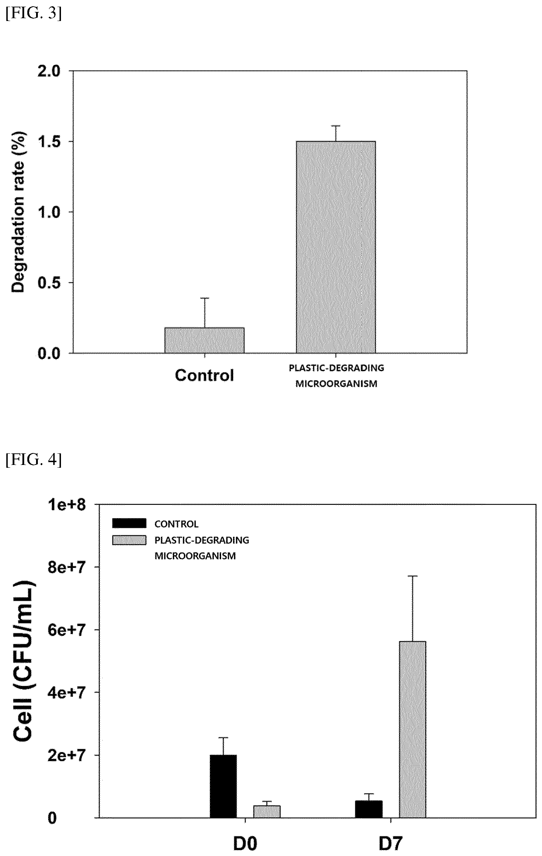

As a result of measuring the plastic degradation rate, it was found that the plastic was degraded when a color change of the redox indicator occurred, but the plastic was not degraded when no color change occurred (FIG. 3).

### 1-3. Comparison of color change of redox indicator and growth of microorganisms

A strain with plastic-degrading activity and a strain without plastic-degrading activity were each cultured under the same conditions as described in Example 1-1. The viable cell count of the strains on day 7 of culture was expressed as colony-forming units (CFUs). Specifically, 1 mL of culture solution was added to 9 mL of minimal medium, and a total of 7 dilutions were made in the same manner, and 100 µL of each dilution was spread on a solid nutrient medium. After culturing in a bioreactor for 24 hours, the number of colonies was measured to determine the viable cell count.

As a result, microorganisms without plastic-degrading activity died in the minimal medium, and the viable cell count decreased, while the viable cell count of microorganisms with plastic-degrading activity increased (FIG. 4). These results mean that microorganisms degraded and metabolized a plastic and used it as an energy source, and therefore, plastic-degrading strains may be identified through the color change of the redox agent.

### Manufacturing Example: Manufacture of a plastic-degrading microorganism identification kit

A minimum medium containing a plastic in powder form and a redox indicator was divided into a well plate used in biotechnology experiments. Then, a single microbial colony was inoculated into each well using a loop and cultured. When the inoculated microorganism was a plastic-degrading strain, a color change occurred.

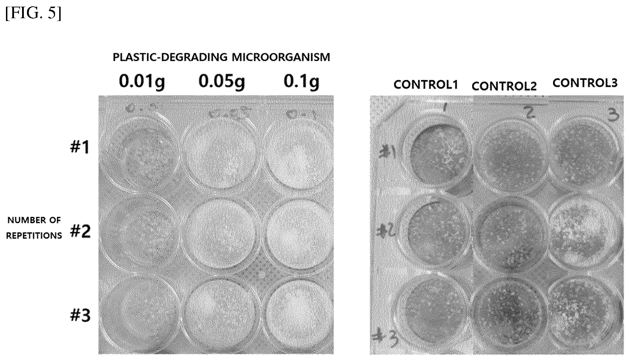

To confirm the effectiveness of the manufactured kit, one strain with plastic-degrading ability and three control strains without plastic-degrading ability were inoculated into the kit for identifying a microorganism with plastic-degrading ability and cultured for seven days. As a result of the culture, the color of the redox indicator changed in the case of the strain with plastic-degrading ability, and the color change became more evident as the amount of plastic increased. However, no color change was observed in the strain without plastic-degrading ability (FIG. 5). In FIG. 5, #1, #2, and #3 indicate the number of repetitions, and reproducibility was confirmed by repeating the experiment three times under the same conditions.

## Claims

1. A method of identifying a microorganism having plastic-degrading activity, comprising:
(a) culturing a microorganism to be tested for plastic-degrading activity in a minimal medium containing a redox indicator and a plastic; and
(b) observing a color change of the redox indicator.

2. The method of claim 1, further comprising (c) determining the microorganism as having plastic-degrading activity when the color of the redox indicator changes.

3. The method of claim 1, wherein the redox indicator is selected from the group consisting of 2,6-dichlorophenolindophenol (DCPIP), methylene green (MG), and thionine.

4. The method of claim 1, wherein the plastic is one or more selected from the group consisting of polyethylene terephthalate (PET), polyvinyl chloride (PVC), polystyrene (PS), polypropylene (PP), polyurethane (PU), and polyethylene (PE).

5. A method of identifying a microorganism having plastic-degrading activity, comprising:
(a) adding a culture medium of a microorganism to be tested for plastic-degrading activity to a minimal medium containing a redox indicator and a plastic;
(b) observing a color change of the redox indicator; and
(c) determining the microorganism as having plastic-degrading activity when the color of the redox indicator changes.

6. The method of claim 5, wherein the redox indicator is selected from the group consisting of 2,6-dichlorophenolindophenol (DCPIP), methylene green (MG), and thionine.

7. The method of claim 5, wherein the plastic is one or more selected from the group consisting of polyethylene terephthalate (PET), polyvinyl chloride (PVC), polystyrene (PS), polypropylene (PP), polyurethane (PU), and polyethylene (PE).

8. A composition for identifying a microorganism having plastic-degrading activity, comprising a redox indicator and plastic powder.

9. A kit for identifying a microorganism having plastic-degrading activity, comprising a redox indicator and plastic powder.
